# EUROPEAN PATENT SPECIFICATION

(11) **EP 1 961 452 B1**
(45) Date of publication and mention of the grant of the patent: **23.10.2013**
(21) Application number: 07117081.5
(22) Date of filing: 24.09.2007
(51) Int. Cl.: A61Q 11/00, A61K 8/02, A61K 8/24

(54) **Oral polyphosphate compositions**
Orale Polyphosphatzusammensetzungen
Compositions orales de polyphosphate

(30) Priority: 23.02.2007 EP 07102987
(43) Date of publication of application: 27.08.2008
(73) Proprietor: The Procter & Gamble Company, Cincinnati, OH 45202 (US)
(72) Inventor: Strand, Ross, Warfield, Middlesex RG42 3RZ (GB); de la Harpe, Shane Michael, Woking, Surrey GU22 9PX (GB); Gunenc, Pelin, Teddington, Middlesex TW11 8BY (GB)
(74) Representative: Joos, Uta Susanne

(56) References cited:
- EP-A- 0 002 184
- EP-A- 0 341 660
- WO-A-02/30382
- WO-A-93/19728
- WO-A-2006/022849
- FR-A- 2 495 467
- US-A- 2 876 167
- US-A- 5 730 959

## Description

### FIELD OF THE INVENTION

The present invention relates to oral compositions comprising water soluble, particulate, long-chain polyphosphate salts.

### BACKGROUND OF THE INVENTION

Water soluble polyphosphates are useful ingredients for oral compositions, such as toothpastes, because they are effective anticalculus agents. They are generally used in their salt form, in particular as sodium polyphosphates.

WO 00/32159 and WO 01/34108 disclose that certain polyphosphates, in particular, linear polyphosphates with average chain lengths of about 4 or more will reduce the staining of the stannous without reducing the efficacy of the stannous.

WO 01/34107 further discloses that polymeric surface active agents, including long chain linear polyphosphates provide novel surface conditioning reactions to oral surfaces such as the teeth and mucosa. This leads to an improved cleaning impression delivered by oral care products or dentifrices containing these agents.

It should be noted that there are also water insoluble polyphosphates which are also used in oral compositions as abrasives; see for example, US 4,272,513 and US 4,780,293.

For the formulator of dentifrices, the water soluble, long chain, linear polyphosphates present some problems through in that they are susceptible to hydrolysis to orthophosphate salts which lack the benefits of the long chain polyphosphates. The problem is especially marked in the presence of fluoride ions. One solution to the problem is to formulate two phase products in which fluoride ions are kept in a physically distinct phase from the polyphosphate salts. Another solution is to formulate the polyphosphate in an anhydrous product or in a product with only a low level of water. In such products the polyphosphate is typically in particulate form. If the polyphosphate particle size is large then the surface area is relatively low and stability to hydrolysis may be acceptable even in the presence of some water. Large particles can create perceptions of grittiness by users of the products though, leading to lowered consumer acceptance. Conversely, if there are too many small particles not only is hydrolysis worsened through the increased surface area but formulating substantial amounts of the polyphosphate into a liquid product can result in unacceptable thickening, especially if there are already high amounts of particulate abrasives in the composition.

It has now been found that by manufacturing a polyphosphate salt to a particular particle size range complaints about grittiness due to its use in a dentifrice can be substantially reduced or avoided without the stability of the particulate polyphosphate towards hydrolysis, or the product rheology, becoming unacceptable.

### SUMMARY OF THE INVENTION

The present invention relates to a dentifrice composition comprising:
a) a polyphosphate salt in particulate form, the polyphosphate salt comprising a polyphosphate anion comprising at least 10 phosphate units; and
b) an orally acceptable carrier;
characterised in that the polyphosphate salt has a particle size distribution wherein less than 10% by volume of the salt has a particle size of greater than 160 µm and less than 20% by volume of the salt has a particle size of less than 5 µm. The dentifrice composition has good stability and acceptable mouth feel.

The invention further relates to orally acceptable polyphosphate salts for use in such dentifrice compositions.

### DETAILED DESCRIPTION OF THE INVENTION

Unless specified otherwise, all percentages and ratios herein are by weight of the total composition and all measurements are made at 25°C.

The term "orally acceptable carrier" as used herein includes any safe and effective materials for use in the compositions of the present invention. Such materials include conventional additives in oral care compositions including but not limited to fluoride ion sources, anticalculus or anti-tartar agents, desensitizing agents, teeth whitening agents such as peroxide sources, abrasives such as silica, herbal agents, chelating agents, buffers, anti-staining agents, alkali metal bicarbonate salts, thickening materials, humectants, water, surfactants, a flavour system, sweetening agents, colouring agents, and mixtures thereof.

The term "dentifrice", as used herein, means a substance for cleaning the teeth which is suitable for application with a toothbrush and is rinsed off after use. It can be a powder, paste, gel, or liquid formulation unless otherwise specified. Dentifrice compositions herein can be single, dual or multi phase preparations. A single phase may comprise a liquid carrier with one or more insoluble particles, such as of a dental abrasive, homogeneously or evenly dispersed within it. The dentifrice compositions herein preferably take the form of tooth pastes and gel.

### Polyphosphate salts

The dentifrice compositions of the present invention comprise a water soluble, linear polyphosphate salt in particulate form, the polyphosphate salt comprising a polyphosphate anion comprising at least 10 phosphate units. Useful levels of the polyphosphate salt are generally greater than 1%, preferably from 1.5% to 50%, more preferably from 2% to 20%, and most preferably from 5% to 15% by weight, of polyphosphate salt.

The polyphosphates are commercially available as salts comprising a polyphosphate anion. It is preferred that the polyphosphate is an alkali metal salt or mixtures thereof, preferably a sodium or potassium salt or mixtures thereof and more preferably a sodium salt. Polyphosphate is a widely used term which refers to phosphate anions which have been polymerised by dehydration to form a polymer of the phosphate anion. Polyphosphates can exist as linear or cyclic materials or mixtures thereof. Preferred polyphosphates herein are linear materials comprising only low levels of cyclic materials. Polyphosphates can also be characterised by the average anion chain length of the polymer anion. For the purposes of this invention the polyphosphates referred to are those water soluble, linear polyphosphate salts with an average anion chain length of 10 or greater. It is preferred that the polyphosphates have an average anion chain length of from 10 to 40, preferably of from 15 to 30; more preferably of 18 to 30, and mixtures thereof.

Polyphosphates with an average anion chain length of greater than four usually occur as glassy material. As defined herein a "glassy" material is one which is amorphous. Preferred in this invention are the linear "glassy" polyphosphates having the formula:

XO(XPO₃)ₙX

wherein X is sodium, potassium, or hydrogen and n averages greater than or equal to 10 or mixtures thereof. Such polyphosphates are manufactured by e.g., FMC Corporation who use the tradenames Hexaphos (n≈13), and Glass H (n≈21), and by Budenheim KG of Rheinstrasse 27, Budenheim, Germany under the Budit® tradename. These polyphosphates may be used alone or in combination. A broad range of phosphates and their sources are described in Kirk-Othmer, Encyclopedia of Chemical Technology, Fourth Edition, Volume 18, Wiley-Interscience Publishers (1996).

The particle size of the polyphosphate salt of the invention can be measured by any competent method capable of yielding a volume distribution of the of particle size. An exemplary method is to use the Malvern Mastersizer® S particle size analyzer, commercially available from Malvern Instruments Ltd., Worcestershire, WR14 1XZ, UK. Other equivalent instruments capable of measuring particles over at least the 0.5 - 900 µm size range may also be used. In the exemplary method the Malvern Mastersizer® S is equipped with a dry powder feeder unit MS64 and a 300F lens (range 0.5 - 900 µm). The instrument should be operated following Malvern standard procedures and guidelines as defined in the equipment manual. The Mastersizer should be switched on for an hour and full laser alignment and background reads should be carried out before use. The experimental setup parameters used are as follows:

| Analysis model | polydisperse |
|---|---|
| Presentation set up | 3RHA Standard dry |
| Sweep Trigger | Set to Internal |
| Experiment trigger | Set to Internal |
| Sweeps sample number | Set at 4000 |
| Sweeps sample time | Set at 8 s |
| Background number | Set at 2000 |
| Sweep Obscuration limits | Measure time out at 99 hrs 59 mins 59 s |
| Upper Limit: | 30% |
| Lower Limit: | 3% |
| Enable: | All |

Measurement data are deemed acceptable if enough sample at a sufficient feed rate has entered the machine to ensure an obscuration read of between 15 - 20%; a particle count of between 3500 - 4000 and a residual of < 1.0%. Background data should be minimised to avoid interfering with the analysis.

Polyphosphate salts according to one embodiment of the invention have a particle size distribution such that less than 5% by volume of the salt has a particle size of greater than 160 µm. Polyphosphate salts according to the invention preferably have a particle size distribution such that less than 10%, more preferably less than 5% by volume of the salt has a particle size of greater than 140 µm. In other embodiments, less than 20%, preferably less than 10%, by volume has a particle size of greater than 100 µm. Also, less than 20% by volume of the salt has a particle size of less than 30 µm.

Alternatively, from 60 to 100%, preferably from 75 to 100%, by volume of the polyphosphate salt has a particle size in the range of from 5 to 140 µm. In preferred embodiments from 60 to 100%, preferably from 70 to 100%, by volume of the polyphosphate salt has a particle size in the range of from 10 to 100 µm.

Commercially available, particulate, food grade polyphosphates suitable for oral use typically comprise proportionately more larger particles than those suitable for the present invention. The particle size distributions desired herein can be obtained by milling or sieving a commercial source of particulate polyphosphate to remove larger particles, and in some instances smaller particles. If necessary, different grades of polyphosphate can be blended together to achieve the desired particle size distribution.

The solubility of the particulate polyphosphate is preferably at least lg per 100ml at 25°C, more preferably at least 5g, even more preferably at least 8g, further more preferably at least 10g, and most preferably at least 15g per 100ml at 25°C. Preferred polyphosphate salts have a pH, in 1% aqueous solution, of from 5.8 to 6.7.

### Other ingredients

The dentifrice compositions of the present invention comprise an orally acceptable carrier which comprises one or more compatible solid or liquid excipients or diluents which are suitable for topical oral administration. By "compatible," as used herein, is meant that the components of the composition are capable of being commingled without interaction in a manner which would substantially reduce the composition's stability and/or efficacy.

The carriers or excipients of the present invention can include the usual and conventional components of toothpastes and gels, as more fully described hereinafter.

The pH of the compositions herein will generally range from 5 to 9.5, more preferably from 5 to 8.5. The pH of a dentifrice composition is measured from a 3:1 aqueous slurry of the dentifrice, i.e., 3 parts water to 1 part dentifrice.

Water is commonly used as a carrier material in dentifrice compositions. It is useful as a processing aid, is benign to the mouth and it assists in quick foaming of toothpastes. Water may be added as an ingredient in its own right or it may be present as a carrier in other common raw materials such as sorbitol and sodium lauryl sulphate. The term 'total water' as used herein means the total amount of water present in the composition, whether added separately or as a solvent or carrier for other raw materials but excluding that which may be present as water of crystallisation in certain inorganic salts. Despite its advantages, the total amount of water in the dentifrice compositions of the present invention should generally be kept low to avoid hydrolysis of the polyphosphate salt. The compositions may be anhydrous. Preferred dentifrice compositions herein are aqueous compositions comprising from 1% to 20%, preferably from 2% to 10%, more preferably from 2% to 5% total water.

Other non-aqueous components of toothpastes and gels generally include one or more of a dental abrasive (from 6% to 50%), a surfactant (from 0.1% to 2.5%), a thickening agent (from 0.1% to 5%), a humectant (from 15% to 45%), a flavouring agent (from 0.04% to 2%), a sweetening agent (from 0.1% to 3 %), a colouring agent (from 0.01% to 0.5%) and water (from 2% to 45%). Such toothpaste or tooth gel may also include one or more of an anticaries agent (typically from 0.05% to 0.25% as fluoride ion) and antimicrobial agents (from 0.1% to 3%).

Types of orally acceptable carriers or excipients which may be included in compositions of the present invention, along with specific non-limiting examples, are discussed in the following paragraphs.

An optional but preferred component of the compositions herein is a humectant. The humectant serves to keep the dentifrice from hardening upon exposure to air, to give a moist feel to the mouth, and, for particular humectants, to impart a desirable sweetness of flavour.

The humectant, on a pure humectant basis, generally comprises from 5% to 70%, preferably from 15% to 45%, by weight of the composition. Suitable humectants include edible polyhydric alcohols such as glycerin, sorbitol, xylitol, butylene glycol, polyethylene glycol, and propylene glycol, especially sorbitol and glycerin.

Dentifrice compositions of the present invention will generally also include a surfactant. Useful surfactant types include anionic, nonionic, cationic and betaine surfactants. Anionic surfactants can be included to provide cleaning and foaming properties, and are typically used in an amount from 0.1% to 2.5%, preferably from 0.3% to 2.5% and most preferably from 0.5% to 2.0% by weight. Cationic surfactants can also be used though care needs to be taken over their compatibility with other ingredients. They would typically be used at levels similar to those of the additional anionic surfactants, as would betaine surfactants. Some nonionic surfactants may be useful at substantially higher levels, such as up to 20% if it is desired to use them to form a ringing gel.

Anionic surfactants useful herein include the water-soluble salts of alkyl sulfates having from 10 to 18 carbon atoms in the alkyl radical and the water-soluble salts of sulfonated monoglycerides of fatty acids having from 10 to 18 carbon atoms. Sodium lauryl sulfate and sodium coconut monoglyceride sulfonates are examples of anionic surfactants of this type. Also useful herein are sarcosinate surfactants, alkyl sulfoacetates, isethionate surfactants and taurate surfactants, such as lauroyl sarcosinate, myristoyl sarcosinate, palmitoyl sarcosinate, stearoyl sarcosinate and oleoyl sarcosinate. All of the foregoing are generally used as their alkali metal or ammonium salts.

Examples of suitable nonionic surfactants include the poloxamers, polyethylene oxide condensates of alkyl phenols, long chain tertiary amine oxides, long chain tertiary phosphine oxides, long chain dialkyl sulfoxides and mixtures of such materials. Preferred betaine surfactants include cocoamidoethyl betaine, cocoamidopropyl betaine, lauramidopropyl betaine and the like.

Cationic surfactants useful in the present invention include derivatives of aliphatic quaternary ammonium compounds having one long alkyl chain containing from 8 to 18 carbon atoms such as lauryl trimethylammonium chloride; cetyl pyridinium chloride; cetyl trimethylammonium bromide; di-isobutylphenoxyethyl-dimethylbenzylammonium chloride; cetyl pyridinium fluoride; etc. Some of these cationic surfactants are also useful as anti-microbial agents.

In preparing toothpaste or gels, it is often necessary to add a thickening agent or binder to provide a desirable consistency of the composition, to provide desirable active release characteristics upon use, to provide shelf stability, and to provide stability of the composition, etc. Thickening agents can include carboxyvinyl polymers, carrageenan, nonionic cellulose derivatives such as hydroxyethyl cellulose, and water soluble salts of cellulose derivatives such as sodium carboxymethylcellulose. Natural gums such as gum karaya, xanthan gum, gum arabic, and gum tragacanth can also be used herein. Xanthan gum is preferred. Suitable thickening agent levels can range from 0.1 to 5%, and higher if necessary.

A preferred ingredient herein for a dentifrice, more especially a toothpaste, is a dental abrasive. Abrasives serve to polish the teeth and /or remove surface deposits. The abrasive material contemplated for use herein can be any material which does not excessively abrade dentine. Suitable abrasives include insoluble phosphate polishing agents, include various calcium phosphates such as, for example, dicalcium phosphate, tricalcium phosphate, calcium pyrophosphate, beta-phase calcium pyrophosphate, dicalcium phosphate dihydrate, anhydrous calcium phosphate, insoluble sodium metaphosphate, and the like. Also suitable are chalk-type abrasives such as calcium and magnesium carbonates, silicas including xerogels, hydrogels, aerogels and precipitates, alumina and hydrates thereof such as alpha alumina trihydrate, aluminosilicates such as calcined aluminium silicate and aluminium silicate, magnesium and zirconium silicates such as magnesium trisilicate and thermosetting polymerised resins such as particulate condensation products of urea and formaldehyde, polymethylmethacrylate, powdered polyethylene and others such as disclosed in US-A-3,070,510, December 25, 1962. Mixtures of abrasives can also be used. Silica dental abrasives of various types are preferred because of their unique benefits of exceptional dental cleaning and polishing performance without unduly abrading tooth enamel or dentine. The abrasive polishing materials generally have an average particle size of from 0.1 to 30 microns, preferably from 5 to 15 microns.

The total amount of abrasive in dentifrice compositions of the subject invention typically range from 6% to 70% by weight; toothpastes preferably contain from 10% to 50%, more preferably from 10% to 30% of abrasives, by weight of the composition. With relatively high levels of abrasives, such as 15% or more, the effect of small particle size polyphosphates on product rheology can be more marked.

Another preferred ingredient is a water-soluble fluoride compound, used in an amount sufficient to give a fluoride ion concentration in the composition of from about 0.01% to 0.35%, preferably from 0.05% to 0.2% by weight, to provide anti-caries effectiveness. A wide variety of fluoride ion-yielding materials can be employed as sources of soluble fluoride in the present compositions. Representative fluoride ion sources include stannous fluoride, sodium fluoride, potassium fluoride, sodium monofluorophosphate and many others. Stannous fluoride and sodium fluoride are particularly preferred, as well as mixtures thereof. To minimise polyphosphate hydrolysis the fluoride ion source can be kept in a separate phase to that containing the polyphosphate salt.

Another optional component of the present compositions is a dentinal desensitizing agent to control hypersensitivity, such as salts of potassium, calcium, strontium and tin including nitrate, chloride, fluoride, phosphates, pyrophosphate, polyphosphate, citrate, oxalate and sulfate salts.

Antimicrobial agents may also be employed. Included among such agents are water insoluble non-cationic antimicrobial agents such as halogenated diphenyl ethers, particularly triclosan and essential oils such as thymol. Water soluble antimicrobials include quaternary ammonium salts such as cetyl pyridinium chloride. Enzymes are another type of active that may be used in the present compositions. Useful enzymes include those that belong to the category of proteases, lytic enzymes, plaque matrix inhibitors and oxidases. The oxidases also have whitening/cleaning activity, in addition to anti-microbial properties. Such agents are disclosed in U.S. Patent 2,946,725, Jul. 26, 1960, to Norris et al. and in U.S. Patent 4,051,234, September 27, 1977 to Gieske et al. Preferred antimicrobial agents also include metal ions such as stannous and zinc ions. Stannous fluoride serves as an antimicrobial agent as well as being a fluoride ion source.

Flavouring and sweetening agents are preferably also included in the present compositions. Suitable flavouring agents and sweetening agents are well known in the art. Suitable flavour levels in the present oral compositions herein are from 0.1% to 5.0%, more preferably from 0.5% to 1.5%, by weight. Typically, a flavour oil will be manufactured in a separate step and will comprise multiple components, natural and/or synthetic in origin, in order to provide a balanced flavour which is acceptable to a broad range of people. Flavour components can be selected from mint, spice, fruit, citrus, herbal, medicinal, and common food flavour types (e.g. chocolate). Illustrative, but non-limiting examples of such components include hydrocarbons such as limonene, caryophyllene, myrcene, and humulene; alcohols such as menthol, linalool, 3-decanol, and pinocarveol; ketones such as piperitone, menthone, spicatone, and 1-carvone; aldehydes such as acetaldehyde, 3-hexanal, or n-octanal; oxides such as menthofuran, piperitone oxide, or carvyl acetate-7,7 oxide; acids such as acetic and ocenoic; and sulphides such as dimethyl sulphide. Components also include esters such as menthyl acetate, benzyl isobutyrate, and 3-octyl acetate. The flavour components may also include essential oils such as peppermint oils from e.g., Mentha piperita and Mentha arvensis; spearmint oils such as those from Mentha cardiaca and Mentha spicata; sage oil, parsley oil, marjoram oil, cassia oil, clove bud oil, cinnamon oil, orange oil, , lime oil, eucalyptus oil and anise oil. Other suitable components are cinnamic aldehyde, eugenol, ionone, anethole, eucalyptol, thymol, methyl salicylate, vanillin, ethyl vanillin, and vanilla extracts. Flavour components are described in more detail in Fenaroli's Handbook of Flavor Ingredients, Third Edition, Volumes 1 & 2, CRC Press, Inc. (1995), and Steffen Arctander's Perfume and Flavour Chemicals, Volumes 1 & 2, (1969). A physiological cooling agent can also be incorporated into the flavour oil. The coolant can be any of a wide variety of materials. Included among such materials are carboxamides, menthol, acetals, ketals, diols, and mixtures thereof. Preferred coolants herein include the p-menthane carboxamide agents such as N-ethyl-p-menthane-3-carboxamide, (known commercially as "WS-3") and mixtures thereof and menthone glycerine acetal (known commercially as "MGA"). Further coolants suitable for the present invention are disclosed in WO 97/06695.

The compositions herein can further include herbal ingredients such as extracts of chamomile, oak bark, melissa, rosemary and salvia. These, and some of the herb-derived flavouring components mentioned above (such as thymol) can be included at levels just sufficient to provide a contribution to the flavour or they can be added at higher levels, such as 1% or more, in order to provide a greater therapeutic effect.

Sweetening agents which can be used include sucrose, glucose, saccharin, sucralose, dextrose, levulose, lactose, mannitol, sorbitol, fructose, maltose, xylitol, saccharin salts, thaumatin, aspartame, D-tryptophan, dihydrochalcones, acesulfame and cyclamate salts, especially sodium cyclamate, sucralose and sodium saccharin, and mixtures thereof. A composition preferably contains from 0.1% to 3% of these agents, more preferably from 0.1% to 1%.

The compositions may further include usual pigments, dyes and opacifiers, such as titanium dioxide. It will be appreciated that selected components for the compositions must be chemically and physically compatible with one another.

The nature of the present invention will be understood with reference to the following non-limiting examples in which Examples 1 - 4 are toothpastes according to the invention and Example C is a comparative example with the same formulation as Example 1 except that the polyphosphate has a larger particle size distribution.

| | 1 | 2 | 3 | 4 | C |
|---|---|---|---|---|---|
| Stannous fluoride | 0.45 | | 0.45 | | 0.45 |
| Sodium monofluorophosphate | | | | 0.8 | |
| Sodium polyphosphate¹ | 13.00¹ | 26.00¹ | 14.00² | 12.0¹ | 13.00³ |
| Silica | 25.00 | 18.15 | 25.00 | | 25.00 |
| Calcium carbonate | | | | 50.0 | |
| Propylene glycol | 7.00 | 3.00 | 7.00 | | 7.00 |
| Polyethylene glycol 300 | 7.00 | | 7.00 | | 7.00 |
| Polyethylene glycol 600 | | 2.75 | | | |
| Polyethylene oxide | | 0.20 | | | |
| Sodium lauryl sulfate, 28% solⁿ. | 4.30 | 8.00 | 3.40 | 7.0 | 4.30 |
| Zinc lactate dihydrate | 2.50 | | 2.50 | | 2.50 |
| Sodium phosphate tribasic, dodecahydrate | 1.10 | | 1.10 | 0.3 | 1.10 |
| Flavour | 1.00 | | 0.60 | 0.9 | 1.00 |
| Sodium gluconate | 0.65 | | 0.65 | | 0.65 |
| Carrageenan | 0.60 | | 0.40 | | 0.60 |
| Sodium saccharin | 0.50 | 0.40 | 0.40 | 0.25 | 0.50 |
| Colour solution (1%) | 0.30 | | 0.30 | | 0.30 |
| Titanium dioxide | | 0.50 | | | |
| CMC sodium | | 0.40 | | 0.6 | |
| Xanthan gum | 0.25 | 0.25 | 0.25 | | 0.25 |
| Sorbitol | | | | 12.0 | |
| Water | | | | to 100% | |
| Glycerin | to 100% | to 100% | to 100% | - | to 100% |

| | | | | | |
|---|---|---|---|---|---|
| ¹ Commercial, food grade, particulate salt sieved to produce a particle size distribution comprising less than 10% by volume greater than 140 µm, less than 3% by volume greater than 160 µm and less than 20% by volume less than 30 µm. ² Commercial, food grade, particulate salt milled to produce a particle size distribution comprising less than 15% by volume greater than 100 µm, less than 10% by volume greater than 120 µm, less than 30% by volume less than 20 µm and less than 10% by volume less than 5 µm. ³ Commercial, food grade, particulate salt having a particle size distribution comprising about 40% by volume greater than 140 µm, about 30% by volume greater than 160 µm and less than 10% by volume less than 30 µm. | | | | | |

Examples 1 and 3 had comparable hydrolytic stability to Comparative Example C but had improved consumer acceptance deriving from a much reduced perception of grittiness.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A dentifrice composition comprising:
a) a water soluble polyphosphate salt in particulate form, the polyphosphate salt comprising a polyphosphate anion comprising at least 10 phosphate units;
b) an orally acceptable carrier;
**characterised in that** the polyphosphate salt has a particle size distribution wherein, less than 5% by volume of the salt has a particle size of greater than 160 µm and less than 10% by volume of the salt has a particle size of greater than 140 µm and less than 20% by volume of the salt has a particle size of less than 30 µm.

2. A dentifrice composition according to Claim 1 in the form of an aqueous toothpaste or gel having a pH of from 5 to 9.5.

3. A dentifrice composition according to Claim 1 or Claim 2 wherein the polyphosphate anion comprises from 18 to 30 phosphate units

4. A dentifrice composition according to Claim 2 comprising from 1% to 20%, preferably from 2% to 10% total water.

5. A dentifrice composition according to Claim 4 comprising sufficient amount of a fluoride ion source to provide from 0.01% to 0.35% fluoride ion by weight of the dentifrice composition.

6. A dentifrice composition according to Claim 5 wherein the fluoride ion source is selected from sodium fluoride, stannous fluoride and mixtures thereof.

7. A dentifrice composition according to any preceding claim wherein less than 20% by volume of the salt has a particle size of greater than 120 µm and wherein less than 20% by volume of the salt has a particle size of less than 20 µm.

## Patentansprüche

1. Zahnreinigungszusammensetzung, umfassend:
a) ein wasserlösliches Polyphosphatsalz in Teilchenform, wobei das Polyphosphatsalz ein Polyphosphatanion umfasst, das mindestens 10 Phosphateinheiten umfasst,
b) einen oral annehmbaren Träger,
**dadurch gekennzeichnet, dass** das Polyphosphatsalz eine Teilchengrößenverteilung aufweist, bei der weniger als 5 Volumen% des Salzes eine Teilchengröße von mehr als 160 µm aufweist und weniger als 10 Volumen% des Salzes eine Teilchengröße von mehr als 140 µm aufweist und weniger als 20 Volumen% des Salzes eine Teilchengröße von weniger als 30 µm aufweist.

2. Zahnreinigungszusammensetzung nach Anspruch 1 in Form einer wässrigen Zahnpasta oder eines Gels mit einem pH-Wert von 5 bis 9,5.

3. Zahnreinigungszusammensetzung nach Anspruch 1 oder Anspruch 2, wobei das Polyphosphatanion 18 bis 30 Phosphateinheiten aufweist.

4. Zahnreinigungszusammensetzung nach Anspruch 2, insgesamt 1% bis 20 %, vorzugsweise 2 % bis 10 % Wasser aufweisend.

5. Zahnreinigungszusammensetzung nach Anspruch 4, eine ausreichende Menge einer Fluoridionenquelle aufweisend, um, bezogen auf das Gewicht der Zahnreinigungszusammensetzung, 0,01 % bis 0,035 % Fluoridionen zu liefern.

6. Zahnreinigungszusammensetzung nach Anspruch 5, wobei die Fluoridionenquelle ausgewählt ist aus Natriumfluorid, Zinnfluorid und Mischungen davon.

7. Zahnreinigungszusammensetzung nach einem der vorstehenden Ansprüche, wobei weniger als 20 Volumen% des Salzes eine Teilchengrößer von mehr als 120 µm aufweist, und wobei weniger als 20 Volumen% des Salzes eine Teilchengröße von weniger als 20 µm aufweist.

## Revendications

1. Composition dentifrice comprenant :
a) un sel polyphosphate hydrosoluble sous forme particulaire, le sel polyphosphate comprenant un anion polyphosphate comprenant au moins 10 motifs phosphate ;
b) un véhicule oralement acceptable ;
**caractérisé en ce que** le sel polyphosphate a une granulométrie dans laquelle, moins de 5% en volume du sel a une taille des particules supérieure à 160 µm et moins de 10 % en volume du sel a une taille des particules supérieure à 140 µm et moins de 20 % en volume du sel a une taille des particules inférieure à 30 µm.

2. Composition dentifrice selon la revendication 1, sous la forme d'une pâte dentifrice aqueuse ou d'un gel ayant un pH allant de 5 à 9,5.

3. Composition dentifrice selon la revendication 1 ou la revendication 2, dans laquelle l'anion polyphosphate comprend de 18 à 30 motifs phosphate

4. Composition dentifrice selon la revendication 2, comprenant de 1 % à 20 %, de préférence de 2 % à 10 % d'eau totale.

5. Composition dentifrice selon la revendication 4, comprenant une quantité suffisante d'une source d'ions fluorure pour fournir de 0,01 % à 0,35 % d'ion fluorure en poids de la composition dentifrice.

6. Composition dentifrice selon la revendication 5, dans laquelle la source d'ions fluorure est choisie parmi le fluorure de sodium, le fluorure stanneux et leurs mélanges.

7. Composition dentifrice selon l'une quelconque des revendications précédentes, dans laquelle moins de 20 % en volume du sel a une taille des particules supérieure à 120 µm et dans laquelle moins de 20 % en volume du sel a une taille des particules inférieure à 20 µm.
